# EUROPÄISCHE PATENTANMELDUNG

(11) **EP 4 212 899 A1**
(43) Veröffentlichungstag der Anmeldung: **19.07.2023**
(21) Anmeldenummer: 22151380.7
(22) Anmeldetag: 13.01.2022
(51) Int. Cl.: G01R 33/36, G01R 33/567

(54) **VERFAHREN ZUR DURCHFÜHRUNG EINER MAGNETRESONANZMESSUNG, EINE MAGNETRESONANZVORRICHTUNG UND EIN COMPUTERPROGRAMMPRODUKT**

(71) Anmelder: Siemens Healthcare GmbH, 91052 Erlangen (DE)
(72) Erfinder: BIBER, Stephan, 91056 Erlangen (DE); SPEIER, Peter, 91056 Erlangen (DE)

(57) **Zusammenfassung**

Die Erfindung betrifft ein Verfahren zur Durchführung einer Magnetresonanzmessung, eine Magnetresonanzvorrichtung und ein Computerprogrammprodukt. Das Verfahren umfasst eine Auswahl einer ersten Menge von Spulenelementen aus der Mehrzahl von Spulenelementen, und einer zweiten Menge von Spulenelementen aus der Mehrzahl von Spulenelementen, ein Durchführen einer Magnetresonanzmessung, wobei während der Magnetresonanzmessung mit der ersten Menge von Spulenelementen und der zweiten Menge von Spulenelementen Magnetresonanzsignale und Pilottonsignale empfangen werden, ein Ermitteln zumindest einer Magnetresonanzabbildung ausschließlich anhand von Magnetresonanzsignalen, die beim Durchführen der Magnetresonanzmessung mit der ersten Menge an Spulenelementen empfangen wurden, und ein Ermitteln einer Bewegungsinformation des Patienten ausschließlich anhand von Pilottonsignalen, die beim Durchführen der Magnetresonanzmessung mit der zweiten Menge an Spulenelementen empfangen wurden, dadurch gekennzeichnet, dass die erste Menge von Spulenelementen nicht deckungsgleich ist mit der zweiten Menge von Spulenelementen.

## Beschreibung

Die Erfindung betrifft ein Verfahren zur Durchführung einer Magnetresonanzmessung, eine Magnetresonanzvorrichtung und ein Computerprogrammprodukt.

In der Medizintechnik zeichnet sich die Bildgebung mittels Magnetresonanz (MR), auch Magnetresonanztomographie (MRT, engl. Magnetic Resonance Imaging, MRI) genannt, durch hohe Weichteilkontraste aus. Bei einer Magnetresonanzmessung eines Patienten werden mit Hilfe einer Magnetresonanzvorrichtung hochfrequente (HF, engl. radio-frequency, RF) Pulse zur Erzeugung eines HF-Feldes und Gradientenpulse zur Erzeugung magnetischer Feldgradienten in einen Untersuchungsbereich, in dem sich der Patient befindet, eingestrahlt. Dadurch werden im Patienten ortskodierte Magnetresonanzsignale ausgelöst.

Die Magnetresonanzsignale werden von der Magnetresonanzvorrichtung empfangen und zur Rekonstruktion von Magnetresonanzabbildungen verwendet. Zum Empfang dieses Magnetresonanzsignals werden vorzugsweise lokale Empfangsspulen, sogenannte Lokalspulen verwendet, die zur Erzielung eines besseren Signal-Rauschverhältnisses (SNR) unmittelbar am Patienten angeordnet werden. Die Lokalspulen weisen üblicherweise eine oder mehrere Spulenelemente auf, die zum Empfang von HF-Signalen, insbesondere Magnetresonanzsignalen, ausgebildet sind.

Die Druckschriften US 10222443 B2 und Speier et al. PT-Nav: A Novel Respiratory Navigation Method for Continuous Acquisition Based on Modulation of a Pilot Tone in the MR-Receiver. ESMRMB, 129:97-98, 2015 offenbaren ein Verfahren, welches es ermöglicht, zeitliche Abläufe während einer Magnetresonanzmessung auf physiologische Bewegungen, wie z.B. Atmung und/oder Herzschlag, zu triggern. Hierdurch können Bewegungsartefakte vermieden (prospektive Bewegungskorrektur) und/oder im Zuge einer digitalen Nachverarbeitung beseitigt werden (retrospektive Bewegungskorrektur).

Dabei wird üblicherweise ein schwaches, hinreichend konstantes, insbesondere hinsichtlich Amplitude und/oder Frequenz, HF-Signal von einem üblicherweise kleinen Pilottonsignal-Generator emittiert. Eine solcher Pilottonsignal-Generator ist beispielhaft in der Druckschrift US 10393845 B2 beschrieben. Das emittierte Signal wechselwirkt mit dem Patienten und wird dann von Spulenelementen einer Empfangsspule, üblicherweise Spulenelementen der Lokalspulen, als Pilottonsignal empfangen. (Eine solche Empfangsspule kann natürlich auch zusätzlich ausgebildet sein, HF-Signale zu senden, d.h. es kann sich auch um eine Sende-Empfangsspule handeln.) Die Empfangsspulen werden, wie oben bereits beschrieben, üblicherweise gleichzeitig für die Bildgebung, also zum Empfang von Magnetresonanzsignalen, genutzt. Eine spektrale Trennung des Pilottonsignals vom Magnetresonanzsignal im empfangenen HF-Signal erlaubt eine störungsfreie Aufnahme von bildgebenden Magnetresonanzsignalen und einen gleichzeitigen Empfang des Pilottonsignals. Amplitude und Phase des Pilottonsignals können dann genutzt werden, um den zeitlichen Verlauf von physiologischen Signalen, wie der Atmung und/oder des Herzschlags, aufzunehmen. Das Magnetresonanzsignale des empfangenen HF-Signals werden dagegen verwendet, um Magnetresonanzabbildungen zu rekonstruieren.

Aus Aufgabe der vorliegenden Erfindung kann angesehen werden, ein verbessertes Verfahren zur Durchführung einer Magnetresonanzmessung eines Patienten anzugeben. Insbesondere soll die Pilotton-Technik noch gezielter eingesetzt werden.

Die Aufgabe wird durch die Merkmale der unabhängigen Ansprüche gelöst. Vorteilhafte Ausgestaltungen sind in den abhängigen Ansprüchen beschrieben.

Demnach wird ein, insbesondere computerimplementiertes, Verfahren zur Durchführung einer Magnetresonanzmessung eines Patienten mittels einer Magnetresonanzvorrichtung vorgeschlagen. Die Magnetresonanzvorrichtung umfasst hierbei eine Mehrzahl von Spulenelementen zum Empfang von HF-Signalen, insbesondere Magnetresonanzsignalen und/oder Pilottonsignalen, umfasst. Es wird eine erste Menge von Spulenelementen aus der Mehrzahl von Spulenelementen ausgewählt, insbesondere um Magnetresonanzsignale zu empfangen. Die erste Menge von Spulenelementen umfasst zumindest ein erstes Spulenelement. Ferner wird eine zweite Menge von Spulenelementen aus der Mehrzahl von Spulenelementen ausgewählt, insbesondere um Pilottonsignale zu empfangen. Die zweite Menge von Spulenelementen umfasst zumindest ein zweites Spulenelement. Dabei ist die erste Menge von Spulenelementen nicht deckungsgleich ist mit der zweiten Menge von Spulenelementen. Jedoch kann die erste Menge von Spulenelementen mit der zweiten Menge an Spulenelementen überlappen. Ferner wird eine Magnetresonanzmessung durchgeführt, wobei während der Magnetresonanzmessung mit der ersten Menge von Spulenelementen und der zweiten Menge von Spulenelementen Magnetresonanzsignale und Pilottonsignale empfangen werden.

Ferner wird zumindest eine Magnetresonanzabbildung ausschließlich anhand von Magnetresonanzsignalen ermittelt, die beim Durchführen der Magnetresonanzmessung mit der ersten Menge an Spulenelementen empfangen wurden. Ferner wird eine Bewegungsinformation des Patienten, insbesondere eine physiologische Information ausschließlich anhand von Pilottonsignalen ermittelt, die beim Durchführen der Magnetresonanzmessung mit der zweiten Menge an Spulenelementen empfangen wurden.

Durch die gezielte Auswahl von Spulenelemente aus der Mehrzahl an Spulenelementen, um aus den mit diesen Spulenelementen empfangenen HF-Signalen, insbesondere Magnetresonanzsignalen, eine oder mehrere Magnetresonanzabbildungen zu ermitteln, sowie durch die gezielte Auswahl von Spulenelemente aus der Mehrzahl an Spulenelementen, um aus den mit diesen Spulenelementen empfangenen HF-Signalen, insbesondere Pilottonsignalen, eine Bewegungsinformationen zu ermitteln, kann die Pilotton-Technik über den bisherigen Anwendungsbereich hinaus nutzbar gemacht werden.

Insbesondere betreffen die ermittelte zumindest eine Magnetresonanzabbildung und die ermittelte Bewegungsinformation unterschiedliche räumliche Bereiche des Patienten, insbesondere des Körpers des Patienten. Vorzugsweise wird die erste Menge von Spulenelementen hinsichtlich ihrer Eignung ausgewählt, Magnetresonanzsignale zu empfangen, um die zumindest eine Magnetresonanzabbildung zu ermittelt. Vorzugsweise wird die zweite Menge von Spulenelementen hinsichtlich ihrer Eignung ausgewählt, Pilottonsignale zu empfangen, um die Bewegungsinformation zu ermittelt. Durch die gezielte Auswahl der jeweiligen Spulenelemente kann beiden Zwecken besser Rechnung getragen werden.

Insbesondere, wenn das Bildgebungsvolumen (also der räumliche Bereich, von dem die zumindest eine Magnetresonanzabbildung ermittelt wird) sich von einem räumlichen Bereich, in dem eine interessierende Bewegung, insbesondere ein physiologischer Vorgang, des Patienten stattfindet, ist eine dedizierte Auswahl der Spulenelemente vorteilhaft. Dies wäre beispielsweise bei einer Messung des Kopfes des Patienten mit Herzschlag- und/oder Atemsynchronisation der Fall. Insbesondere können durch eine gezielte Auswahl der Spulenelemente hinsichtlich ihres Zweckes vorteilhafterweise Bildartefakte vermieden werden, die dadurch entstehen, dass Signale in die Rekonstruktion der zumindest einen Magnetresonanzabbildung einfließen, die aus Bereichen, insbesondere weit, außerhalb des Bildgebungsvolumen herrühren.

Statt eine (physiologische) Bewegung im Bildgebungsvolumen zu erfassen, kann sie vorteilhafterweise an einer anderen Stelle gemessen werden, wo sie leichter detektierbar ist. Dies soll am Beispiel einer Kopfuntersuchung demonstriert werden: In großen Gefäßen ist der Blutfluss üblicherweise pulsatil. Vorteilhaft für die Aufnahme der Magnetresonanzsignale ist eine Synchronisation mit dem Flusszustand des Blutes. Der Fluss wird durch die Pumparbeit des Herzens erzeugt. Durch Beobachten der Herzbewegung mit dem Pilottonsignalen kann daher diese Synchronisation erreicht werden, da der Blutkreislauf die vom Pilottonsignal detektierte Bewegung mit einer Bewegung im Bildgebungsvolumen verbindet.

Ein anderes Anwendungsbeispiel sind Magnetresonanzmessungen, bei denen sich eine Frequenz der Magnetresonanz im Kopf abhängig von der Atembewegung ändert. Dabei kann genutzt werden, dass die vom Pilottonsignal detektierte Atembewegung direkt die Resonanzfrequenz in der Umgebung, also auch im Kopf, ändert.

Ferner kann die ermittelte Bewegungsinformation, insbesondere durch ein Bedienpersonal der Magnetresonanzvorrichtung, beispielsweise dazu verwendet werden, um den Zustand, insbesondere des Patienten während der Magnetresonanzmessung zu überwachen. Beispielsweise kann anhand der Bewegungsinformation, insbesondere anhand der Atmung, auf einen Zustand des Patienten geschlossen werden, z.B. ob der Patient nervös ist oder schläft. Somit kann das vorgeschlagene Verfahren auf effiziente Weise insbesondere auch Untersuchungen ermöglicht werden, bei denen die physiologischen Parameter nicht für die MR-Aufnahme an sich genutzt werden (z.B. in Form einer Bewegungskorrektur der Magnetresonanzsignale), sondern um beispielsweise festzustellen, wie sich der Patient gerade fühlt.

Zur Atemüberwachung ist es besonders vorteilhaft, Pilottonsignale aus dem Bereich des Abdomens zu erfassen. Wenn nun beispielsweise Magnetresonanzabbildungen des Kopfes oder des Knies des Patienten ermittelt werden, so liegt das Bildgebungsvolumen relativ weit entfernt zum Abdomen. Durch jeweils eine eigene Menge an Spulenelemente (also die erste Menge und die zweite Menge, wobei sich diese Mengen durchaus überlappen können) kann sowohl eine optimale Bildgebung als auch eine optimale Überwachung physiologischer Parameter erreicht werden.

Vorteilhafterweise wird das vorgeschlagene Verfahren bei Untersuchungen eingesetzt, bei denen die Atem- oder Herzschlagsynchronisation zur Stabilisierung des Ergebnisses beiträgt oder die Messung erst ermöglicht, obwohl der Brustkorb sich nicht im Bildgebungsvolumen befindet. Dazu zählen beispielsweise funktionelle Magnetresonanztomographie (fMRT, engl. fMRI) am Kopf des Patienten oder kontrastmittelfreie Magnetresonanzangiographie wie z.B. wie Quiescent-Inflow Single-Shot (QISS) oder Non-contrast MRA of ArTerIes and VEins (NATIVE) in den Beinen.

Zur Erzeugung von Pilottonsignalen werden vorzugsweise HF-Signale mit einem Pilottonsignal-Generator erzeugt, die mit dem Patienten wechselwirken. Ein das Pilottonsignal empfangendes Spulenelement kann insbesondere ein Teil einer Spule, insbesondere einer Lokalspule und/oder einer fest in die Magnetresonanzvorrichtung eingebaute Körperspule, sein. Ein das Pilottonsignal empfangendes Spulenelement ist vorzugsweise ausgelegt, das empfangene Pilottonsignal zum Ermitteln der Bewegungsinformation des Patienten, insbesondere zur Auswertung einer Information über einen physiologischen Vorgang in dem Patienten und/oder einer Bewegung des Patienten an eine Systemsteuereinheit der Magnetresonanzvorrichtung zu übertragen.

Der Pilottonsignal-Generator kann beispielsweise Teil einer Lokalspule sein, insbesondere in einer Lokalspule integriert und/oder eingebaut sein. Vorteilhafterweise kann dadurch der Pilottonsignal-Generator besonders nahe am Patienten positioniert werden, so dass die durch die Spulenelemente empfangenen Pilottonsignale vorteilhafterweise ein besonders hohes SNR aufweisen.

Das durch den Pilottonsignal-Generator generierte HF-Signal weist vorzugsweise ein erstes Frequenzband auf, wobei das empfangende Spulenelement dazu ausgebildet ist, ein Empfangs-Frequenzband aufzunehmen, das das erste Frequenzband umfasst. Vorzugsweise umfasst die Magnetresonanzvorrichtung eine Hochfrequenzantenneneinheit, die ausgebildet ist, einen HF-Puls mit einem zweiten Frequenzband auszugegeben. Die empfangenden Spulenelemente sind vorzugsweise dazu ausgebildet, ein Magnetresonanzsignal des HF-Pulses zu empfangen, wobei das Magnetresonanzsignal ein drittes Frequenzband aufweist, das zumindest im Wesentlichen außerhalb des ersten Frequenzbandes liegt. Vorteilhafterweise kollidiert das erste Frequenzband nicht mit dem eigentlichen Messsignal, dem Magnetresonanzsignal, das im dritten Frequenzband liegt.

Die Auswahl der ersten Menge von Spulenelementen aus der Mehrzahl von Spulenelementen und/oder die Auswahl der zweiten Menge von Spulenelementen aus der Mehrzahl von Spulenelementen können beispielsweise mittels einer Systemsteuereinheit der Magnetresonanzvorrichtung erfolgen. Die Systemsteuereinheit kann insbesondere einen oder mehrere Prozessoren und/oder ein oder mehrere Speichermodule umfassen.

Die erste Menge von Spulenelementen und die zweite Menge von Spulenelementen können eine Vereinigungsmenge bilden. Vorzugsweise ist die Anzahl an Elementen, insbesondere Spulenelementen, dieser Vereinigungsmenge kleiner oder gleich einer maximal zur Verfügung stehenden Anzahl an Empfangskanälen der Magnetresonanzvorrichtung, wobei jeder der Empfangskanäle jeweils ausgebildet ist, das von einem Spulenelement empfangene HF-Signal zu empfangen und insbesondere an eine Systemsteuereinheit der Magnetresonanzvorrichtung weiterzuleiten. Das empfangene HF-Signal kann insbesondere ein Magnetresonanzsignal und/oder ein Pilottonsignal umfassen.

Beispielsweise weist die Magnetresonanzvorrichtung 16 Spulenkanäle auf; die erste Menge von Spulenelementen weist zehn Spulenelemente auf; die zweite Menge von Spulenelementen weist acht Spulenelemente auf; zwei Spulenelemente sind sowohl ein Teil der ersten Menge von Spulenelementen und der zweiten Menge von Spulenelementen, d.h. auch anhand der durch diese beiden Spulenelemente empfangenen Magnetresonanzsignale und Pilottonsignale werden zumindest eine Magnetresonanzabbildung und eine Bewegungsinformation des Patienten ermittelt; somit umfasst die Vereinigungsmenge 16 Spulenelemente; jedes der 16 Spulenelemente kann mit jeweils einem Empfangskanal der Magnetresonanzvorrichtung ausgelesen werden.

Das Durchführen der Magnetresonanzmessung kann insbesondere eine Aussendung von HF-Pulsen und Gradientenpulsen gemäß einer vorgegebenen Magnetresonanzsequenz umfassen. Die HF-Pulse können insbesondere Anregungspulse und/oder Refokussierungspulse umfassen. Die Gradientenpulse können insbesondere Schichtselektionsgradientenpulse, Phasenkodiergradientenpulse und/oder Auslesegradientenpulse umfassen.

Das Ermitteln der zumindest einen Magnetresonanzabbildung kann insbesondere kann insbesondere eine Rekonstruktion der Magnetresonanzsignale umfassen, die (ausschließlich) durch die Spulenelemente der ersten Menge von Spulenelementen empfangen wurde. Das Ermitteln der Bewegungsinformation des Patienten kann insbesondere die Auswertung der Pilottonsignalen umfassen, die (ausschließlich) durch die Spulenelemente der zweiten Menge von Spulenelementen empfangen wurde.

Dass die erste Menge von Spulenelementen nicht deckungsgleich ist mit der zweiten Menge von Spulenelementen, kann insbesondere bedeuten, dass ein erster Teil der Vereinigungsmenge der ersten Menge von Spulenelementen und der zweiten Menge von Spulenelementen Spulenelemente sind, die nur zur ersten Menge von Spulenelementen gehören, dass ein zweiter Teil der Vereinigungsmenge der ersten Menge von Spulenelementen und der zweiten Menge von Spulenelementen Spulenelemente sind, die nur zur zweiten Menge von Spulenelementen gehören, und dass ein dritter Teil der Vereinigungsmenge der ersten Menge von Spulenelementen und der zweiten Menge von Spulenelementen Spulenelemente sind, die sowohl zur ersten Menge von Spulenelementen als auch zur zweiten Menge von Spulenelementen Spulenelemente erhören. Der dritte Teil der Vereinigungsmenge stellt also die Schnittmenge der ersten Menge von Spulenelementen und der zweiten Menge von Spulenelementen dar. Es ist auch denkbar, dass die Schnittmenge eine leere Menge ist, d.h. dass es keine Spulenelemente gibt, deren empfangene HF-Signale sowohl zum Ermitteln der zumindest einer Magnetresonanzabbildung als auch zum Ermitteln der Bewegungsinformation genutzt wird.

Die Mächtigkeit (also die Anzahl der Elemente der jeweiligen Menge) der ersten Menge von Spulenelementen und der zweiten Menge von Spulenelementen beträgt vorzugsweise zumindest eins, d.h. sowohl die erste Menge von Spulenelementen umfasst vorzugsweise zumindest ein Spulenelement als auch die zweite Menge von Spulenelementen umfasst vorzugsweise zumindest ein Spulenelement.

Vorzugsweise erfolgt die Auswahl der ersten Menge von Spulenelementen (zum Empfang von Magnetresonanzsignalen) vor der Auswahl der zweiten Menge von Spulenelementen (zum Empfang von Pilottonsignalen). Vorzugsweise wird die erste Menge von Spulenelementen zuerst ausgewählt und im Anschluss daran wird die zweite Menge von Spulenelementen ausgewählt. Vorzugsweise erfolgt die Auswahl der zweiten Menge von Spulenelementen in Abhängigkeit von der ersten Menge der Spulenelemente. Vorzugsweise wird die erste Menge von Spulenelemente mit einer höheren Priorität ausgewählt als die zweite Menge von Spulenelementen. Insbesondere können zunächst die Spulenelemente ausgewählt werden, die sich besonders für die Bildgebung eignen, und darauffolgend die Spulenelemente, die sich besonders zur Erfassung des Bewegungssignals eignen. Diese Reihenfolge der Auswahl kann insbesondere dann von Vorteil sein, wenn die Qualität der zumindest einen Magnetresonanzabbildung gegenüber der Qualität der Bewegungsinformation besonders wichtig ist.

Insbesondere legt die Mächtigkeit der ersten Menge von Spulenelementen und eine maximale Anzahl von Empfangskanälen der Magnetresonanzvorrichtung eine maximale Anzahl an Spulenelementen fest, die zur zweiten Menge von Spulenelemente gehören, aber nicht zur ersten Menge. Umfasst die Magnetresonanzvorrichtung beispielsweise M Empfangskanäle und weist die erste Menge von Spulenelementen N Spulenelemente auf, kann die zweite Menge von Spulenelementen maximal L = M-N Spulenelemente aufweisen, die nicht zur ersten Menge von Spulenelementen gehört. Es ist insbesondere denkbar, dass die Mächtigkeit der zweiten Menge von Spulenelementen bis zu M beträgt, d.h. dass auch bis zu alle Spulenelementen der ersten Menge von Spulenelementen auch zur zweiten Menge von Spulenelementen gehört.

Es ist auch denkbar, dass die Auswahl der zweiten Menge von Spulenelementen (zum Empfang von Pilottonsignalen) vor der Auswahl der ersten Menge von Spulenelementen (zum Empfang von Magnetresonanzsignalen) erfolgt. Vorzugsweise wird die zweite Menge von Spulenelementen zuerst ausgewählt und im Anschluss daran wird die erste Menge von Spulenelementen ausgewählt. Vorzugsweise erfolgt die Auswahl der ersten Menge von Spulenelementen in Abhängigkeit von der zweiten Menge der Spulenelemente. Vorzugsweise wird die zweite Menge von Spulenelemente mit einer höheren Priorität ausgewählt als die erste Menge von Spulenelementen. Insbesondere können zunächst die Spulenelemente ausgewählt werden, die sich besonders zur Erfassung des Bewegungssignals eignen, und darauffolgend die Spulenelemente, die sich besonders für die Bildgebung eignen. Diese Reihenfolge der Auswahl kann insbesondere dann von Vorteil sein, wenn die Qualität der Bewegungsinformation gegenüber der Qualität der zumindest einen Magnetresonanzabbildung besonders wichtig ist.

Insbesondere legt die Mächtigkeit der zweiten Menge von Spulenelementen und eine maximale Anzahl von Empfangskanälen der Magnetresonanzvorrichtung eine maximale Anzahl an Spulenelementen fest, die zur ersten Menge von Spulenelemente gehören, aber nicht zur zweiten Menge. Umfasst die Magnetresonanzvorrichtung beispielsweise M Empfangskanäle und weist die zweite Menge von Spulenelementen L Spulenelemente auf, kann die erste Menge von Spulenelementen maximal N=M-L Spulenelemente aufweisen, die nicht zur zweiten Menge von Spulenelementen gehört. Es ist insbesondere denkbar, dass die Mächtigkeit der ersten Menge von Spulenelementen bis zu M beträgt, d.h. dass auch bis zu alle Spulenelementen der zweiten Menge von Spulenelementen auch zur ersten Menge von Spulenelementen gehört.

Denkbar ist auch, dass die erste Menge von Spulenelementen (zum Empfang von Magnetresonanzsignalen) und die zweite Menge von Spulenelementen (zum Empfang von Pilottonsignalen) gemeinsam ausgewählt wird. Vorzugsweise wird die erste Menge von Spulenelementen parallel zur zweiten Menge von Spulenelementen ausgewählt. Vorzugsweise erfolgt die Auswahl der ersten Menge von Spulenelementen und der zweiten Menge von Spulenelementen in gegenseitiger Abhängigkeit. Vorzugsweise wird die erste Menge von Spulenelemente mit einer gleichen Priorität ausgewählt wie die zweite Menge von Spulenelementen. Insbesondere können die Spulenelemente, die sich besonders für die Bildgebung eignen, und die Spulenelemente, die sich besonders zur Erfassung des Bewegungssignals eignen, gemeinsam ausgewählt werden. Diese Reihenfolge der Auswahl kann insbesondere dann von Vorteil sein, wenn die Qualität der zumindest einen Magnetresonanzabbildung gegenüber der Qualität der Bewegungsinformation ähnlich wichtig ist.

Vorzugsweise kann ein Gewichtungsfaktor festgelegt werden, der eine relative Priorisierung einer voraussichtlichen Qualität der zumindest einen zu ermittelnden Magnetresonanzabbildung gegenüber der Qualität der zu ermittelnden Bewegungsinformation beschreibt. Anhand dieses Gewichtungsfaktors kann die gemeinsame Auswahl der ersten Menge von Spulenelementen und der zweiten Menge von Spulenelementen, insbesondere vollautomatisch und/oder halbautomatisch erfolgen.

Insbesondere legt die Anzahl der Empfangskanäle der Magnetresonanzvorrichtung eine maximale Mächtigkeit der Vereinigungsmenge der ersten Menge von Spulenelementen und der zweiten Menge von Spulenelementen fest. Insbesondere ist die Anzahl der verschiedenen Spulenelemente, die zur ersten Menge von Spulenelementen und zur zweiten Menge von Spulenelementen gehören, kleiner oder gleich der Anzahl der Empfangskanäle der Magnetresonanzvorrichtung.

Vorzugsweise wird zumindest für einen Teil der Mehrzahl von Spulenelementen der Magnetresonanzvorrichtung ihre Position relativ zur Magnetresonanzvorrichtung und/oder relativ zum Patienten ermittelt. Dabei erfolgt die Auswahl der ersten Menge von Spulenelementen (zum Empfang von Magnetresonanzsignalen) und/oder der zweiten Menge von Spulenelementen (zum Empfang von Pilottonsignalen) abhängig von der jeweils ermittelten Position.

Vorteilhafterweise kann bei bekannter Position der Spulenelemente besser abgeschätzt werden, ob bzw. in welchem Maße die das jeweilige Spulenelemente geeignet ist, Magnetresonanzsignale zum Ermitteln der zumindest einen Magnetresonanzabbildung und/oder Pilottonsignale zum Ermitteln der Bewegungsinformation des Patienten zu ermitteln.

Befindet sich ein Spulenelement beispielsweise in der Nähe des Herzens des Patienten, kann es besonders gute Pilottonsignale zur Ermittlung des Herzschlags des Patienten empfangen. Befindet sich ein Spulenelement beispielsweise in der Nähe des Abdomens des Patienten, kann es besonders gute, insbesondere ein hohes SNR aufweisende, Pilottonsignale zur Ermittlung der Atembewegung des Patienten empfangen.

Vorzugsweise erfolgt die Auswahl der ersten Menge von Spulenelementen (zum Empfang von Magnetresonanzsignalen) und/oder der zweiten Menge von Spulenelementen (zum Empfang von Pilottonsignalen) ferner abhängig von einer Art bzw. Technik der Magnetresonanzmessung und/oder von einem zu untersuchenden Bereich des Patienten.

Ist der untersuchende Bereich des Patienten beispielsweise der Kopf des Patienten und befindet sich ein Spulenelement beispielsweise in der Nähe des Kopfes des Patienten, kann das Spulenelement besonders gute, insbesondere ein hohes SNR aufweisende, Magnetresonanzsignale zur Ermittlung der zumindest einen Magnetresonanzabbildung empfangen.

Ist Art bzw. Technik der Magnetresonanzmessung beispielsweise eine fMRT oder eine kontrastmittelfreie Magnetresonanzangiographie, ist es vorteilhaft den Herzschlag und/oder die Atembewegung des Patienten zu ermitteln. Daher werden vorzugsweise Spulenelemente ausgewählt die nach am Herzen und/oder am Abdomen des Patienten positioniert sind.

Vorzugsweise erfolgt die Ermittlung der Positionen der Spulenelemente mittels einer Auswertung einer gespeicherten spulenspezifischen Information, die eine Position zumindest eines Spulenelements relativ zur Empfangsspule beschreibt, welche das zumindest eine Spulenelement umfasst und/oder einer Auswertung von Magnetresonanzsignalen, die bei einer Justagemessung mit den Spulenelementen empfangen wurden, und/oder einer Auswertung eines Videosignals, das mit zumindest einer Kamera aufgenommen wurde, und/oder einer Auswertung eines Sensorsignals, das mit zumindest einem Positionssensor, z.B. Hall-Signal, RFID-Sensor, erfasst wurde.

Die gespeicherte spulenspezifischen Information kann beispielsweise eine relative Position (beispielsweise in Form von Koordinaten oder einer Information "innen" bzw. "außen") eines Spulenelements innerhalb der Empfangsspule umfassen.

Die Justagemessung kann insbesondere vor der Magnetresonanzmessung durchgeführt werden. Vorteilhafterweise dauert eine solche Justagemessung nur eine kurze Zeitdauer, z.B. nur wenige Sekunden. Vorzugsweise werden bei der Auswertung Magnetresonanzsignale der Justagemessung zur Ermittung der Positionen der Spulenelemente Amplituden und/oder Phasen der Magnetresonanzsignale ausgewertet.

Die Kamera zur Aufnahme des Videosignals kann insbesondere eine 2D-Kamera und/oder eine 3D-Kamera umfassen. Der Sensor zur Erfassung des Sensorsignals kann insbesondere einen Hall-Sensor und/oder einen RFID-Sensor umfassen.

Vorzugsweise kann anhand des Videosignals und/oder des Sensorsignals eine Position einer Empfangsspule relativ zum Patienten ermittelt werden. Vorteilhafterweise können anhand der Position einer Empfangsspule relativ zum Patienten in Kombination mit der gespeicherten spulenspezifischen Information die Positionen der Spulenelemente der Empfangsspule relativ zum Patienten ermittelt werden.

Vorzugsweise erfolgen die Auswahl der ersten Menge von Spulenelementen (zum Empfang von Magnetresonanzsignalen) und/oder der zweiten Menge von Spulenelementen (zum Empfang von Pilottonsignalen) vollautomatisch und/oder halbautomatisch und/oder manuell.

Beispielsweise erfolgen die Auswahl der ersten Menge von Spulenelementen und der zweiten Menge von Spulenelementen vollautomatisch. Beispielsweise erfolgen die Auswahl der ersten Menge von Spulenelementen vollautomatisch und die Auswahl der zweiten Menge von Spulenelementen vollautomatisch. Beispielsweise erfolgen die Auswahl der ersten Menge von Spulenelementen vollautomatisch und die Auswahl der zweiten Menge von Spulenelementen halbautomatisch. Beispielsweise erfolgen die Auswahl der ersten Menge von Spulenelementen halbautomatisch und die Auswahl der zweiten Menge von Spulenelementen halbautomatisch. Beispielsweise erfolgen die Auswahl der ersten Menge von Spulenelementen manuell und die Auswahl der zweiten Menge von Spulenelementen vollautomatisch. Beispielsweise erfolgen die Auswahl der ersten Menge von Spulenelementen manuell und die Auswahl der zweiten Menge von Spulenelementen halbautomatisch. Beispielsweise erfolgen die Auswahl der ersten Menge von Spulenelementen manuell und die Auswahl der zweiten Menge von Spulenelementen manuell. Beispielsweise erfolgen die Auswahl der ersten Menge von Spulenelementen vollautomatisch und die Auswahl der zweiten Menge von Spulenelementen manuell. Beispielsweise erfolgen die Auswahl der ersten Menge von Spulenelementen halbautomatisch und die Auswahl der zweiten Menge von Spulenelementen manuell.

Vorzugsweise wirkt bei der vollautomatischen Auswahl von Spulenelementen ein Bedienpersonal der Magnetresonanzvorrichtung an der Auswahl der Spulenelemente nicht mit. Vorzugsweise wird die vollautomatische Auswahl allein durch die Systemsteuereinheit der Magnetresonanzvorrichtung durchgeführt. Vorteilhafterweise kann eine vollautomatische Auswahl sehr schnell und/oder sehr komfortabel durchgeführt werden.

Vorzugsweise wird bei der halbautomatischen Auswahl dem Bedienpersonal eine Menge von Spulenelementen automatisch vorgeschlagen wird und das Bedienpersonal wählt basierend auf diesem Vorschlag die Spulenelemente, insbesondere manuell, aus. Vorteilhafterweise kann eine halbautomatische Auswahl schnell, komfortabel und/oder besonders sicher durchgeführt werden, da das Bedienpersonal eine Eingriffsmöglichkeit hat.

Eine manuelle Auswahl der Spulenelemente kann beispielsweise dem Bedienpersonal auf einem Bildschirm die zur Verfügung stehenden Spulenelemente angezeigt werden, die das Bedienpersonal, insbesondere manuell, anwählen bzw. abwählen kann. Vorteilhafterweise kann eine manuelle Auswahl flexibel durchgeführt und/oder einfach implementiert werden.

Vorzugsweise erfolgt die Auswahl, insbesondere die vollautomatische Auswahl und/oder der automatische Vorschlag der halbautomatischen Auswahl, der zweiten Menge an Spulenelementen anhand eines Typs zumindest einer Empfangsspule, welche zumindest einen Teil der Mehrzahl von Spulenelementen der Magnetresonanzvorrichtung umfasst, und/oder einer relativen Lage der Spulenelemente innerhalb zumindest einer Empfangsspule, welche zumindest einen Teil der Mehrzahl von Spulenelementen der Magnetresonanzvorrichtung umfasst, und/oder zumindest einer Eigenschaft des Patienten, z.B. Größe, Gewicht, Geschlecht, und/oder einer Positionierung des Patienten in der Magnetresonanzvorrichtung, insbesondere auf der Patientenliege.

Vorzugsweise wird der Typ der zumindest einen Empfangsspule dadurch gekennzeichnet, dass die Empfangsspule zur Messung einer bestimmten Köperregion des Patienten ausgebildet ist (beispielsweise eine Wirbelsäulenspule und/oder eine flexible lokale Empfangsspule, die sich besonders gut am Abdomen des Patienten platzieren lässt) und/oder einen Pilotton-SignalGenerator aufweist.

Vorzugsweise liegen die auszuwählenden Spulenelemente für die zweite Menge an Spulenelementen innerhalb zumindest einer Empfangsspule möglichst weit innen.

Die zumindest eine Eigenschaft des Patienten kann vorzugsweise einer Registrierungsinformationen entnommen werden. Die Registrierungsinformationen wird vorzugsweise durch eine Registrierung des Patienten vor der Durchführung des Magnetresonanzmessung erfasst.

Positionierung des Patienten in der Magnetresonanzvorrichtung kann insbesondere derart ausgestaltet sein, dass der Patient mit dem Kopf voran (engl. head first) oder mit den Füßen voran (engl. feet first) auf der Patientenliege positioniert wird.

Vorzugsweise werden, insbesondere zur vollautomatischen Auswahl und/oder zum automatischen Vorschlag der halbautomatischen Auswahl der zweiten Menge von Spulenelementen, mit zumindest einem Teil der Mehrzahl der Spulenelementen der Magnetresonanzvorrichtung jeweils ein Justage-Pilottonsignal aufgenommen. Dabei werden die Justage-Pilottonsignale hinsichtlich ihres jeweiligen Beitrags zur Ermittlung der Bewegungsinformation des Patienten, insbesondere seines Herzensschlags und/oder seiner Atembewegung, ausgewertet. Ferner wird eine maximale Anzahl K der Spulenelemente der zweiten Menge von Spulenelementen ermittelt wird, wobei K Spulenelemente ausgewählt und/oder vorgeschlagen werden, die den größten Beitrag zur Ermittlung der Bewegungsinformation des Patienten aufweisen.

Die maximale Anzahl K der Spulenelemente kann insbesondere einer Anzahl L an Empfangskanälen entsprechen, die nach einer Auswahl der ersten Menge an Spulenelementen (deren empfangene Magnetresonanzsignale zur Ermittlung der zumindest einen Magnetresonanzabbildung verwendet werden) noch zur Verfügung stehen (insbesondere zur Aufnahme von Pilottonsignalen).

Vorzugsweise wird der zumindest eine Teil der Mehrzahl der Spulenelemente der Magnetresonanzvorrichtung, mit dem jeweils ein Justage-Pilottonsignal aufgenommen wird, bestimmt anhand eines Typs zumindest einer Empfangsspule, welche zumindest einen Teil der Mehrzahl von Spulenelementen der Magnetresonanzvorrichtung umfasst, und/oder einer relativen Lage der Spulenelemente innerhalb zumindest einer Spule, welche zumindest einen Teil der Mehrzahl von Spulenelementen der Magnetresonanzvorrichtung umfasst, und/oder zumindest einer Eigenschaft des Patienten und/oder einer Positionierung des Patienten in der Magnetresonanzvorrichtung, insbesondere auf der Patientenliege.

Insbesondere kann die Bestimmung des zumindest einen Teils der Mehrzahl der Spulenelemente der Magnetresonanzvorrichtung anhand der gleichen Kriterien erfolgen, wie die mögliche Auswahl der zweiten Menge an Spulenelementen, die vorab beschrieben wurde. Vorteilhafterweise werden die besten Kandidaten an Spulenelementen bestimmt, mit denen jeweils ein Justage-Pilottonsignal aufgenommen werden soll. Dadurch kann möglicherweise zeitaufwändige Justagemessung mit allen möglichen Spulenelementen der Magnetresonanzvorrichtung vermieden werden.

Eine weitere Ausführungsform sieht vor, dass zur, insbesondere halbautomatischen und/oder manuellen, Auswahl der Spulenelemente einem Bedienpersonal ein Avatar des Patienten und dazu lagerichtig zumindest ein Teil der Mehrzahl von Spulenelementen der Magnetresonanzvorrichtung angezeigt wird, wobei ferner angezeigt wird, ob ein angezeigtes Spulenelementen zur ersten Menge von Spulenelementen und/oder zur zweiten Menge von Spulenelemente zugeordnet ist.

Vorteilhafterweise kann das Bedienpersonal mit Hilfe dieser Darstellung eine manuelle Auswahl der ersten Menge von Spulenelementen und der zweiten Menge von Spulenelementen durchführen. Eine solche Auswahl kann besonders übersichtlich und komfortabel erfolgen.

Vorzugsweise wird dem Bedienpersonal zur Auswahl der Spulenelemente ferner ein Bildgebungsbereich angezeigt. Der Bildgebungsbereich ist insbesondere ein Bereich des Patienten, wobei die in diesem Bereich erzeugten Magnetresonanzsignale mit den Spulenelementen der ersten Menge von Spulenelementen vorteilhafterweise mit möglichst hoher Qualität, insbesondere möglichst hohem SNR, empfangen werden. Vorteilhafterweise wählt das Bedienpersonal Spulenelemente für die erste Menge an Spulenelemente, die im Bildgebungsbereich und/oder möglichst nahe am Bildgebungsbereich liegen.

Vorteilhafterweise werden, insbesondere im Rahmen einer halbautomatischen Auswahl, dem Bedienpersonal automatisch die Spulenelemente vorgeschlagen, die im Bildgebungsbereich und/oder bis zu einem vorbestimmten Abstand zum Bildgebungsbereich liegen. Das Bedienpersonal kann die Auswahl bestätigen oder abändern.

Ferner wird eine Magnetresonanzvorrichtung mit einer Mehrzahl von Spulenelementen vorgeschlagen, die ausgebildet die vorangehend beschriebenen Ausführungsformen des Verfahrens zur Durchführung einer Magnetresonanzmessung eines Patienten auszuführen.

Vorzugsweise weist die Magnetresonanzvorrichtung eine Systemsteuereinheit zur Auswahl einer ersten Menge von Spulenelementen aus der Mehrzahl von Spulenelementen und einer zweiten Menge von Spulenelementen aus der Mehrzahl von Spulenelementen auf. Die Spulenelemente sind vorzugsweise ausgebildet, während einer Magnetresonanzmessung Magnetresonanzsignale und/oder Pilottonsignale zu empfangen.

Die Systemsteuereinheit ist vorzugsweise ausgebildet, zumindest eine Magnetresonanzabbildung ausschließlich anhand von Magnetresonanzsignalen, die beim Durchführen der Magnetresonanzmessung mit der ersten Menge an Spulenelementen empfangen wurden, zu ermitteln. Außerdem ist die Systemsteuereinheit ist vorzugsweise ausgebildet, eine Bewegungsinformation des Patienten ausschließlich anhand von Pilottonsignalen, die beim Durchführen der Magnetresonanzmessung ausschließlich mit der ersten Menge an Spulenelementen empfangen wurden, zu ermitteln. Dabei ist die erste Menge von Spulenelementen nicht deckungsgleich mit der zweiten Menge von Spulenelementen, d.h. es gibt zumindest ein ausgewähltes Spulenelement, das nicht zu beiden Mengen gehört.

Die Vorteile der erfindungsgemäßen Magnetresonanzvorrichtung entsprechen im Wesentlichen den Vorteilen des erfindungsgemäßen Verfahrens zur Durchführung einer Magnetresonanzmessung eines Patienten mittels einer Magnetresonanzvorrichtung, welche vorab im Detail ausgeführt sind. Hierbei erwähnte Merkmale, Vorteile oder alternative Ausführungsformen können ebenso auch auf die anderen beanspruchten Gegenstände übertragen werden und umgekehrt.

Ferner wird ein Computerprogrammprodukt vorgeschlagen, das ein Programm umfasst und direkt in einen Speicher einer programmierbaren Systemsteuereinheit einer Magnetresonanzvorrichtung ladbar ist und Programmmittel, z.B. Bibliotheken und Hilfsfunktionen, aufweist, um ein erfindungsgemäßes Verfahren auszuführen, wenn das Computerprogrammprodukt in der Systemsteuereinheit der Magnetresonanzvorrichtung ausgeführt wird.

Das Computerprogrammprodukt kann dabei eine Software mit einen Quellcode, der noch kompiliert und gebunden oder der nur interpretiert werden muss, oder einen ausführbaren Softwarecode umfassen, der zur Ausführung nur noch in die Systemsteuereinheit zu laden ist. Durch das Computerprogrammprodukt kann das erfindungsgemäße Verfahren schnell, identisch wiederholbar und robust ausgeführt werden. Das Computerprogrammprodukt ist so konfiguriert, dass es mittels der Systemsteuereinheit die erfindungsgemäßen Verfahrensschritte ausführen kann. Die Systemsteuereinheit umfasst dabei jeweils die Voraussetzungen wie beispielsweise einen entsprechenden Arbeitsspeicher, eine entsprechende Grafikkarte oder eine entsprechende Logikeinheit, so dass die jeweiligen Verfahrensschritte effizient ausgeführt werden können.

Das Computerprogrammprodukt ist beispielsweise auf einem computerlesbaren Medium gespeichert oder auf einem Netzwerk oder Server hinterlegt, von wo es in den Prozessor einer lokalen Systemsteuereinheit geladen werden kann, der mit der Magnetresonanzvorrichtung direkt verbunden oder als Teil der Magnetresonanzvorrichtung ausgebildet sein kann. Weiterhin können Steuerinformationen des Computerprogrammprodukts auf einem elektronisch lesbaren Datenträger gespeichert sein. Die Steuerinformationen des elektronisch lesbaren Datenträgers können derart ausgestaltet sein, dass sie bei Verwendung des Datenträgers in einer Systemsteuereinheit einer Magnetresonanzvorrichtung ein erfindungsgemäßes Verfahren durchführen. Beispiele für elektronische lesbare Datenträger sind eine DVD, ein Magnetband oder einen USB-Stick, auf welchem elektronisch lesbare Steuerinformationen, insbesondere Software, gespeichert ist. Wenn diese Steuerinformationen von dem Datenträger gelesen und in eine Systemsteuereinheit der Magnetresonanzvorrichtung gespeichert werden, können alle erfindungsgemäßen Ausführungsformen der vorab beschriebenen Verfahren durchgeführt werden. So kann die Erfindung auch von dem besagten computerlesbaren Medium und/oder dem besagten elektronisch lesbaren Datenträger ausgehen.

Weitere Vorteile, Merkmale und Einzelheiten der Erfindung ergeben sich aus den im Folgenden beschriebenen Ausführungsbeispielen sowie anhand der Zeichnungen. Einander entsprechende Teile sind in allen Figuren mit den gleichen Bezugszeichen versehen.

Es zeigen:
- Fig. 1: eine Magnetresonanzvorrichtung in einer schematischen Darstellung,
- Fig. 2: ein Blockdiagramm eines Verfahrens Verfahren zur Durchführung einer Magnetresonanzmessung,
- Fig. 3: eine Anzeige zur Auswahl von Spulenelementen.

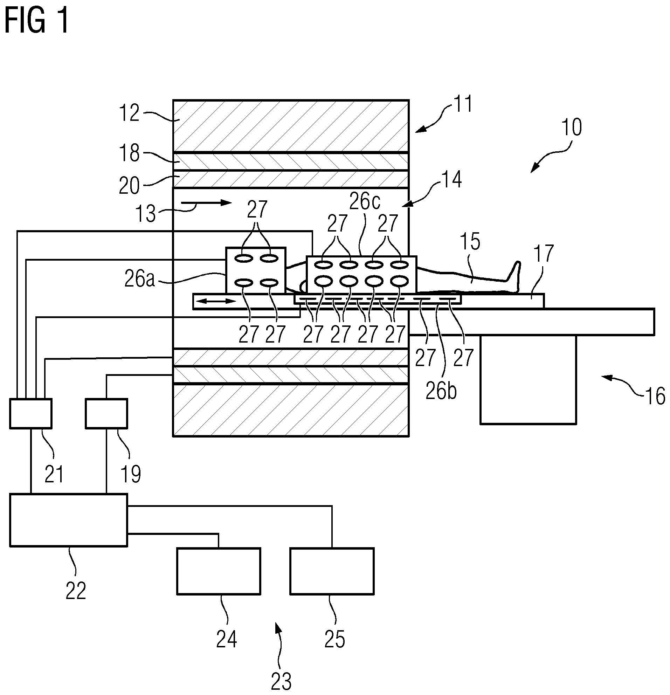

In Fig. 1 ist eine Magnetresonanzvorrichtung 10 schematisch dargestellt. Die Magnetresonanzvorrichtung 10 umfasst eine Magneteinheit 11, die einen Hauptmagneten 12 zu einem Erzeugen eines starken und insbesondere zeitlich konstanten Hauptmagnetfelds 13 aufweist. Zudem umfasst die Magnetresonanzvorrichtung 10 einen Patientenaufnahmebereich 14 zu einer Aufnahme eines Patienten 15. Der Patientenaufnahmebereich 14 im vorliegenden Ausführungsbeispiel ist zylinderförmig ausgebildet und in einer Umfangsrichtung von der Magneteinheit 11 zylinderförmig umgeben. Grundsätzlich ist jedoch eine davon abweichende Ausbildung des Patientenaufnahmebereichs 14 jederzeit denkbar. Der Patient 15 kann mittels einer Patientenlagerungsvorrichtung 16 der Magnetresonanzvorrichtung 10 in den Patientenaufnahmebereich 14 geschoben werden. Die Patientenlagerungsvorrichtung 16 weist hierzu einen innerhalb des Patientenaufnahmebereichs 14 bewegbar ausgestalteten Patiententisch 17 auf.

Die Magneteinheit 11 weist weiterhin eine Gradientenspuleneinheit 18 zu einer Erzeugung von Magnetfeldgradienten auf, die für eine Ortskodierung während einer Bildgebung verwendet werden. Die Gradientenspuleneinheit 18 wird mittels einer Gradientensteuereinheit 19 der Magnetresonanzvorrichtung 10 gesteuert. Die Magneteinheit 11 umfasst weiterhin eine Hochfrequenzantenneneinheit 20, welche im vorliegenden Ausführungsbeispiel als fest in die Magnetresonanzvorrichtung 10 integrierte Körperspule ausgebildet ist. Die Hochfrequenzantenneneinheit 20 wird von einer Hochfrequenzantennensteuereinheit 21 der Magnetresonanzvorrichtung 10 gesteuert und während strahlt einer Magnetresonanzmessung hochfrequente Pulse gemäß einer vorbestimmten Magnetresonanzsequenz in einen Untersuchungsraum, insbesondere in einen Bildgebungsbereich FOV, ein. Dadurch stellt sich dem von dem Hauptmagneten 12 erzeugten Hauptmagnetfeld 13 eine Anregung von Atomkernen ein. Durch Relaxation der angeregten Atomkerne werden Magnetresonanzsignale erzeugt. Die Hochfrequenzantenneneinheit 20 ist zum Empfang der Magnetresonanzsignale ausgebildet.

Zu einer Steuerung des Hauptmagneten 12, der Gradientensteuereinheit 19 und zur Steuerung der Hochfrequenzantennensteuereinheit 21 weist die Magnetresonanzvorrichtung 10 eine Systemsteuereinheit 22 auf. Die Systemsteuereinheit 22 steuert zentral die Magnetresonanzvorrichtung 10, wie beispielsweise das Durchführen einer vorbestimmten bildgebenden Magnetresonanzsequenz. Zudem umfasst die Systemsteuereinheit 22 eine nicht näher dargestellte Systemsteuereinheit zu einer Auswertung der Magnetresonanzsignale, die während der Magnetresonanzuntersuchung erfasst werden. Des Weiteren umfasst die Magnetresonanzvorrichtung 10 eine Benutzerschnittstelle 23, die mit der Systemsteuereinheit 22 verbunden ist. Steuerinformationen wie beispielsweise Bildgebungsparameter, sowie rekonstruierte Magnetresonanzabbildungen können auf einer Anzeigeeinheit 24, beispielsweise auf einem Bildschirm, der Benutzerschnittstelle 23 für ein medizinisches Bedienpersonal angezeigt werden. Weiterhin weist die Benutzerschnittstelle 23 eine Eingabeeinheit 25 auf, mittels der Informationen und/oder Parameter während eines Messvorgangs von dem medizinischen Bedienpersonal eingegeben werden können.

Die Magnetresonanzvorrichtung umfasst ferner drei Empfangsspulen 23a, 23b, 23c, die unmittelbar am Patienten 15 angeordnet sind. Bei den Empfangsspulen 23a, 23b, 23c handelt es sich also um Lokalspulen. Jede der drei Empfangsspulen 23a, 23b, 23c umfasst wiederum mehrere Spulenelemente 27, die üblicherweise in der jeweiligen Empfangsspule 23a, 23b, 23c integriert ist. Die Spulenelemente sind in der Lage, HF-Signale zu empfangen, insbesondere Magnetresonanzsignale und/oder Pilottonsignale. Die empfangenen Signale können an die Systemsteuereinheit 22 übermittelt werden. Anhand der Magnetresonanzsignale können Magnetresonanzabbildungen des Patienten 15 durch die Systemsteuereinheit 22 rekonstruiert werden. Anhand der Pilottonsignale können insbesondere eine Information über Bewegungen des Patienten 15 durch die Systemsteuereinheit 22 ermittelt werden. Dazu umfasst beispielsweise der Empfangsspule 23c einen (hier nicht dargestellten) Pilottonsignal-Generator, der ein HF-Signal aussendet. Dieses HF-Signal tritt in Wechselwirkung mit dem Patienten 15. Insbesondere wird das HF-Signal durch eine Bewegung des Patienten 15 beeinflusst. Insbesondere kann das HF-Signal je nach dem Bewegungszustand des Patienten 15 eine unterschiedliche Amplitude und/oder Phase aufweisen. Das HF-Signal wird als Pilottonsignal durch die Spulenelemente 27 empfangen und an die Systemsteuereinheit 22 zur Auswertung, insbesondere zur Ermittlung einer Bewegungsinformation, weitergeleitet.

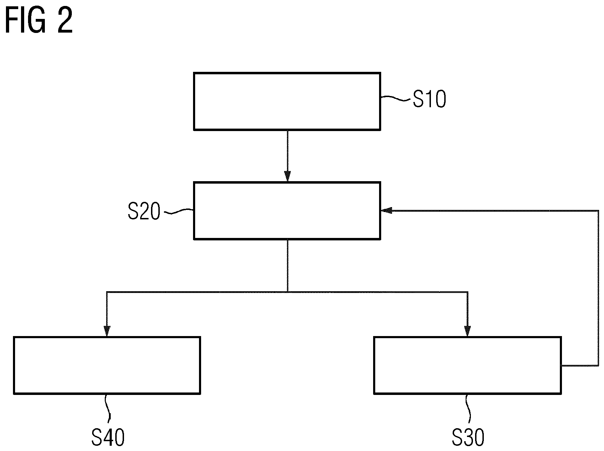

In Fig. 2 ist ein mögliches Verfahren zur Durchführung einer Magnetresonanzmessung des Patienten 15 mittels der Magnetresonanzvorrichtung 10 dargestellt.

In S10 werden eine erste Menge A und eine zweite Menge B von Spulenelementen aus der Mehrzahl von Spulenelementen 27 ausgewählt. Die erste Menge A ist dabei nicht deckungsgleich ist mit der zweiten Menge B, d.h. A≠B. Die Auswahl kann, insbesondere mit Hilfe der Systemsteuereinheit 22, vollautomatisch, halbautomatisch und/oder manuell erfolgen.

In S20 wird eine Magnetresonanzmessung durchgeführt, wobei während der Magnetresonanzmessung mit der ersten Menge und der zweiten Menge von Spulenelementen, insbesondere mit der Vereinigungsmenge der ersten und zweiten Menge AUB, Magnetresonanzsignale und Pilottonsignale empfangen.

In S30 wird eine Bewegungsinformation des Patienten 15 ausschließlich anhand von Pilottonsignalen, die beim Durchführen der Magnetresonanzmessung in S20 mit der zweiten Menge an Spulenelementen B empfangen wurden. Etwaige Pilottonsignale, die mit Spulenelementen der Differenzmenge A\B empfangen wurden, werden also nicht zur Ermittlung der Bewegungsinformation verwendet.

Die Ermittlung der Bewegungsinformation kann insbesondere während der Magnetresonanzmessung erfolgen. Die ermittelte Bewegungsinformation kann insbesondere für eine Steuerung der Magnetresonanzmessung verwendet werden, insbesondere für eine prospektive Bewegungskorrektur. Dabei kann eine der Magnetresonanzmessung zugrunde liegende Magnetresonanzsequenz in Echtzeit und/oder on-the-fly angepasst werden, indem beispielsweise eine Lage einer aufzunehmenden Schicht, insbesondere durch die Systemsteuereinheit 22, angepasst wird.

In S40 wird zumindest eine Magnetresonanzabbildung des Patienten 15 ausschließlich anhand von Magnetresonanzsignalen, die beim Durchführen der Magnetresonanzmessung in S20 mit der zweiten Menge an Spulenelementen B empfangen wurden. Etwaige Pilottonsignale, die mit Spulenelementen der Differenzmenge B\A empfangen wurden, werden also nicht zur Ermittlung der zumindest einen Magnetresonanzabbildung verwendet.

In Folgenden werden insbesondere mögliche Ausführungsformen der Auswahl der ersten Menge A und zweiten Menge B nähe erläutert. Gemäß einer ersten Ausführungsform erfolgt zunächst Auswahl der ersten Menge A, die für die Bildgebung verwendet werden soll, vor der Auswahl der zweiten Menge B. Die erste Menge A kann insbesondere manuell durch das Bedienpersonal der Magnetresonanzvorrichtung 10 oder automatisch erfolgen.

Beispielsweise werden dabei N Spulenelemente aus der Mehrzahl von Spulenelementen ausgewählt. Bei einer beispielhaften Magnetresonanzvorrichtung mit M Empfangskanälen bleiben also L=M-N Kanäle Empfangskanälen für andere Anwendungen als der Bildgebung, wie beispielsweise den Empfang dedizierter Pilottonsignale.

Die Auswahl der zweiten Menge B kann insbesondere vollautomatisch - insbesondere "versteckt", also für das Bedienpersonal unbemerkt - erfolgen. Die Auswahl der zweiten Menge B sowie die Ermittlung der Bewegungsinformation kann insbesondere ausgelöst werden, indem das Bedienpersonal, beispielsweise Rahmen einer Einstellung der Magnetresonanzmessung, eine Atmungsüberwachung oder Triggerung aktiviert.

Die Magnetresonanzvorrichtung erkennt vorzugsweise selbständig, welche der Spulenelemente 27 für die Atmungsüberwachung besonders günstig sind. Dazu können in einem vorgeschalteten Justageschritt Justage-Daten, insbesondere Justage-Pilottonsignale, von potenziell günstigen Empfangsspulen aufgenommen werden.

Potenziell günstige Empfangsspulen sind beispielsweise Spulen, die im Bereich des Abdomens und/oder Thorax des Patienten angeordnet sind. Typischerweise sind das dedizierte Empfangsspulen, so dass die Auswahl der zweiten Menge B auch anhand des Typs zumindest einer Empfangsspule erfolgen kann.

Bevorzugt werden Spulenelemente bestimmter Spulentypen ausgewählt, die im Abdomen-Thorax-Bereich liegen verwendet, z.B. eine Wirbelsäulenspule und/oder eine Body-Matrix-Spule.

Vorzugsweise wird eine relative Lage der Empfangsspulen 26a, 26b, 26c, insbesondere der Spulenelemente 27 der Empfangsspulen 26a, 26b, 26c, ermittelt. Dabei sind in Rechts-links-Richtung innen liegende Spulenelemente wichtiger als die äußeren. Auswahl der zweiten Menge kann also insbesondere von einer relativen Lage der Spulenelemente innerhalb zumindest einer Empfangsspule erfolgen.

Insbesondere können Vorinformationen aus einer Registrierung des Patienten 15 genutzt werden, um zu ermitteln, wo vermutlich die Spulenelemente mit dem höchsten Signalbeitrag liegen könnten. Solche Vorinformationen können beispielsweise eine (geplante) Positionierung des Patienten 15 in der Magnetresonanzvorrichtung 10 umfassen, ob der Patienten mit dem Kopf oder mit den Füßen voran in den Patientenaufnahmebereich 14 geschoben wird. Ferner können die Vorinformationen beispielsweise zumindest Eigenschaft des Patienten 15 umfassen, insbesondere dessen Größe, Gewicht und/oder Geschlecht.

Wenn die Magnetresonanzvorrichtung über eine ausreichende Anzahl an Empfangskanälen verfügt, können Justage-Daten von allen (relevanten) Spulenelementen aufgenommen. Wenn nicht, kann eine Vorauswahl der vorangehend beschriebenen Informationen und/oder Kriterien erstellt werden. Die Elemente können auch in mehreren Gruppen zeitlich nacheinander oder zeitlich verschachtelt (engl. interleaved) aufgenommen werden.

Die Justage-Daten werden, (z.B. mit einer Hauptkomponentenanalyse) untersucht, wie groß der Beitrag der einzelnen Spulenelemente zur Detektion der Bewegungsinformation, z.B. eines Atmungssignals, ist. Dadurch kann eine Rangfolge der besonders geeigneten Spulenelemente erstellt werden.

Die ersten L Spulenelemente aus dieser Auswahl können beispielsweise von der Magnetresonanzvorrichtung 10 zum Empfang von Pilottonsignalen ausgewählt werden, ohne dass die erste Menge A, umfassend die Spulenelemente für die Bildgebung, beschränkt wird. Neben den ersten L Spulenelemente kann die zweite Menge B insbesondere auch geeignete Spulenelemente aus der ersten Menge A umfassen. Insbesondere kann eine maximale Anzahl K der Spulenelemente der zweiten Menge B ermittelt werden, so dass neben den ersten L Spulenelementen noch weitere K-L Spulenelemente aus der ersten Menge A hinzugewählt werden können.

Wenn L sehr klein oder Null ist oder kein ausreichend starkes Signal für eine Bewegung, z.B. eine Atmung, gefunden wurde, kann dem Bedienpersonal insbesondere angezeigt werden, dass die pilottonbasierte Überwachung deaktiviert wurde.

Ein bei der Magnetresonanzmessung in S20 verwendetes Spulenelement kann also nur zur Menge A, nur zur Menge B oder zu beiden Mengen gehören. Insbesondere wird der Hardware der Magnetresonanzvorrichtung 10 zur Durchführung der Magnetresonanzmessung die Information übermittelt, welche Spulenelemente ausgewählt, d.h. die Vereinigungsmenge AUB der Mengen A und B. Die Software der Magnetresonanzvorrichtung 10 kann insbesondere unterscheiden, zu welchem Zweck ein Spulenelement ausgewählt wurde, d.h. ob ein Spulenelement nur zur Menge A, nur zur Menge B oder zur Menge AUB gehört.

Insbesondere können dem Bedienpersonal durch die Anzeigeeinheit 24 nur die Spulenelemente der Menge A für die Bildgebung angezeigt werden, nicht aber die Spulenelemente der Menge B zur Bewegungserfassung.

Gemäß einer weiteren Ausführungsform erfolgt die Auswahl der zweiten Menge B manuell durch das Bedienpersonal. Dazu werden dem Bedienpersonal beispielweise über die Anzeigeeinheit 24 mögliche Spulenelemente angezeigt, die es, beispielsweise durch Anklicken, der zweiten Menge B zuordnen kann. Vorzugsweise ist diese Auswahlmöglichkeit so gestaltet, dass es für das Bedienpersonal klar ist, dass mit den empfangenen Signalen der auszuwählenden Spulenelementen keine Bildrekonstruktion erfolgen soll, sondern dass sie nur zum Ermitteln der Bewegungsinformation anhand der durch sie empfangenen Pilottonsignale dienen.

Gemäß einer weiteren Ausführungsform wird die zweite Menge B von Spulenelementen, insbesondere bei Aktivieren einer Pilottonfunktionalität, vollautomatisch ausgewählt. Insbesondere können die vollautomatisch ausgewählten Spulenelemente vom Bedienpersonal nicht wieder abgewählt werden. Das Bedienpersonal kann dann, soweit es die Anzahl der verfügbaren Empfangskanäle der Magnetresonanzvorrichtung erlaubt, zusätzliche Spulenelemente zur Bildgebung manuell auswählen. Alternativ werden die Spulenelemente zur Bildgebung vollautomatisch ausgewählt, insbesondere auf Grundlage einer Bildgebungsgeometrie. Die Auswahl der zweiten Menge von Spulenelementen erfolgt hier also vor der Auswahl der ersten Menge von Spulenelementen.

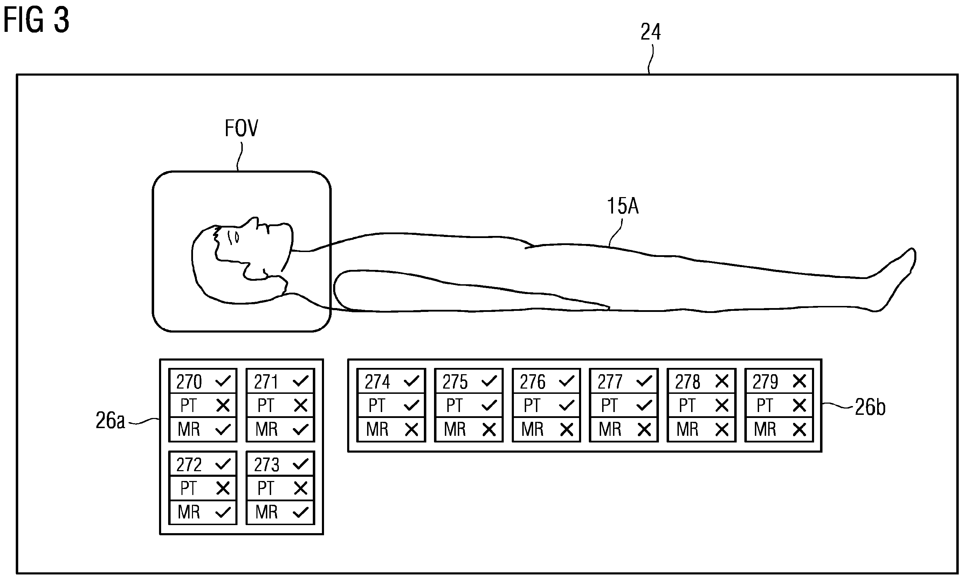

Fig. 3 zeigt beispielhaft eine Darstellung, die dem Bedienpersonal bei der Auswahl der Spulenelemente durch die Anzeigeeinheit 24 angezeigt wird. Dabei werden ein Patientenavatar 15A und dazu lagerichtig Empfangsspulen 26a, 26b mit ihren Spulenelementen 270-279 angezeigt, die bei der folgenden Magnetresonanzmessung verwendet werden können. Die Empfangsspule 26a, die hier eine Kopfspule ist, umfasst die Spulenelemente 270, 271, 272, 273; die Empfangsspule 26a, die hier eine Wirbelsäulenspule ist, umfasst die Spulenelemente 274, 275, 276, 277, 278, 279. Die Lage der Empfangsspulen 26a, 26b bzw. deren Spulenelemente 270-279 kann insbesondere durch eine Justagemessung ermittelt werden, die der eigentlichen Magnetresonanzmessung vorausgeht und bei der üblicherweise Magnetresonanzsignale ausgewertet werden, um die Lage zu bestimmen. Ferner können im Falle ortsfester Empfangsspulen, wie etwa typischerweise einer Kopfspule, die Positionen der Spulenelemente in einer spulenspezifischen Datei abgespeichert sein. Ferner können zur Bestimmung der Lage der Empfangsspulen und/oder der Spulenelemente beispielsweise 2D- und/oder 3D-Kameras und/oder an den Empfangsspulen angebrachte Sensoren, z.B. Hall-Sensoren, eingesetzt werden.

Ferner angezeigt wird, ob ein angezeigtes Spulenelementen zur ersten Menge von Spulenelementen und/oder zur zweiten Menge von Spulenelemente zugeordnet ist. Insbesondere wird dargestellt, welche Spulenelemente zur zweiten Menge B gehören, d.h. für die Ermittlung der Bewegungsinformation vorgesehen sind, und welche zur ersten Menge A, d.h. für die Bildgebung vorgesehen sind. Falls ein Spulenelement zur ersten Menge A gehört, ist das durch "MR" in Verbindung mit einem Häkchen dargestellt; falls ein Spulenelement zur zweiten Menge B gehört, ist das durch "PT" in Verbindung mit einem Häkchen dargestellt. Falls ein Spulenelement nicht zur ersten Menge A gehört, ist das durch "MR" in Verbindung mit einem Kreuzchen dargestellt; falls ein Spulenelement nicht zur zweiten Menge B gehört, ist das durch "PT" in Verbindung mit einem Kreuzchen dargestellt. Falls ein Spulenelement zur Vereinigungsmenge AUB gehört, also überhaupt HF-Signale empfangen soll, ist das durch ihre Spulennummer in Verbindung mit einem Häkchen dargestellt; anderenfalls ist das mit einem Kreuzchen dargestellt. Im dargestellten Fall gehören die Spulenelemente 270, 271, 272, 273 zur ersten Menge A; die durch sie empfangenen Magnetresonanzsignale sind besonders gut geeignet, um daraus zumindest eine Magnetresonanzabbildung des Kopfes des Patienten 15 zu ermitteln. Die Spulenelemente 274, 275, 276, 277 gehören zur zweiten Menge B; die durch sie empfangenen Pilottonsignale sind besonders gut geeignet, um daraus die Bewegung des Herzens und/oder des Abdomens des Patienten 15 zu detektieren. Die Spulenelemente 278, 279 werden hingegen bei der Magnetresonanzmessung nicht verwendet.

Die Auswahl der Spulenelemente kann insbesondere anhand einer Eigenschaft des angezeigten Spulenelements geschehen, insbesondere durch seine Lage: Wird die Anzeige nun durch die Darstellung eines Bildgebungsbereichs FOV, insbesondere eines Bildgebungsvolumens, ergänzt, wird sofort klar, welche Spulenelement zu welchem Zweck geeignet sind. Vor allem die Spulenelemente, die sich innerhalb des Bildgebungsbereichs FOV oder nahe am Bildgebungsbereich FOV befinden, tragen zum Bild bei. Das Kriterium der Position der Empfangsspulen relativ zum Bildgebungsbereich FOV kann auch nach Durchführung der Magnetresonanzmessung zur Entfernung von Spulenelementen aus der ersten Menge A führen, d.h. die Magnetresonanzsignale, die mit solchen entfernten Spulenelementen empfangen wurden, werden dann nicht zum Ermitteln der zumindest einen Magnetresonanzabbildung verwendet.

Vorteilhafterweise werden die Spulenelemente anhand geometrischer Kriterien ausgewählt. Zusätzlich oder alternativ könnten die Spulensymbole außerhalb des Bildgebungsbereichs FOV auch modifiziert werden, um anzuzeigen, dass es sich nicht um Bildgebungsspulenelemente der Menge A handelt. Die Modifikation könnte auf eine Schnittstelle zum Bedienpersonal beschränkt werde; in anderen Teilen der Magnetresonanzvorrichtung 10 müssten vorteilhafterweise die beiden Spulenarten nicht unterschieden werden.

Zusammenfassend lässt sich festhalten, dass das vorgeschlagene Verfahren eine pilottonbasierte Überwachung auch in Körperbereichen möglich macht, in denen Spulenelemente nicht zur Bildgebung verwendet werden, z.B. bei einer Messung des Kopfes des Patienten 15 mit Herzschlag- und/oder Atemsynchronisation. Vorteilhafterweise braucht das Bedienpersonal solche Spulenelemente nicht selbst anwählen.

Es wird abschließend noch einmal darauf hingewiesen, dass es sich bei den vorhergehend detailliert beschriebenen Verfahren sowie bei der dargestellten Magnetresonanzvorrichtung lediglich um Ausführungsbeispiele handelt, welche vom Fachmann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vorhanden sein können. Ebenso schließt der Begriff "Einheit" nicht aus, dass die betreffenden Komponenten aus mehreren zusammenwirkenden Teil-Komponenten bestehen, die gegebenenfalls auch räumlich verteilt sein können.

## Patentansprüche

1. Verfahren zur Durchführung einer Magnetresonanzmessung eines Patienten mittels einer Magnetresonanzvorrichtung, die eine Mehrzahl von Spulenelementen zum Empfang von HF-Signalen umfasst, wobei das Verfahren umfasst:
• Auswahl
o einer ersten Menge von Spulenelementen aus der Mehrzahl von Spulenelementen, und
o einer zweiten Menge von Spulenelementen aus der Mehrzahl von Spulenelementen,
• Durchführen einer Magnetresonanzmessung,
wobei während der Magnetresonanzmessung mit der ersten Menge von Spulenelementen und der zweiten Menge von Spulenelementen Magnetresonanzsignale und Pilottonsignale empfangen werden,
• Ermitteln zumindest einer Magnetresonanzabbildung ausschließlich anhand von Magnetresonanzsignalen, die beim Durchführen der Magnetresonanzmessung mit der ersten Menge an Spulenelementen empfangen wurden,
• Ermitteln einer Bewegungsinformation des Patienten ausschließlich anhand von Pilottonsignalen, die beim Durchführen der Magnetresonanzmessung mit der zweiten Menge an Spulenelementen empfangen wurden,
**dadurch gekennzeichnet, dass** die erste Menge von Spulenelementen nicht deckungsgleich ist mit der zweiten Menge von Spulenelementen.

2. Verfahren nach Anspruch 1,
wobei die Auswahl der ersten Menge von Spulenelementen vor der Auswahl der zweiten Menge von Spulenelementen erfolgt.

3. Verfahren nach Anspruch 1,
wobei die Auswahl der zweiten Menge von Spulenelementen vor der Auswahl der ersten Menge von Spulenelementen erfolgt.

4. Verfahren nach Anspruch 1,
wobei die erste Menge von Spulenelementen und die zweite Menge von Spulenelementen gemeinsam ausgewählt wird.

5. Verfahren nach Anspruch 4,
wobei ein Gewichtungsfaktor festgelegt wird, der eine relative Priorisierung einer voraussichtlichen Qualität der zumindest einen zu ermittelnden Magnetresonanzabbildung gegenüber einer voraussichtlichen Qualität der zu ermittelnden Bewegungsinformation beschreibt,
wobei die gemeinsame Auswahl der ersten Menge von Spulenelementen und der zweiten Menge von Spulenelementen anhand des Gewichtungsfaktors erfolgt.

6. Verfahren nach einem der vorangehenden Ansprüche,
wobei zumindest für einen Teil der Mehrzahl von Spulenelementen der Magnetresonanzvorrichtung ihre Position relativ zur Magnetresonanzvorrichtung und/oder relativ zum Patienten ermittelt wird,
wobei die Auswahl der ersten Menge von Spulenelementen und/oder der zweiten Menge von Spulenelementen abhängig von der jeweils ermittelten Position erfolgt.

7. Verfahren nach Anspruch 6,
wobei die Ermittlung der Positionen der Spulenelemente erfolgt mittels
• einer Auswertung einer gespeicherten spulenspezifischen Information, die eine Position zumindest eines Spulenelements relativ zur Empfangsspule beschreibt, welche das zumindest eine Spulenelement umfasst und/oder
• einer Auswertung von Magnetresonanzsignalen, die bei einer Justagemessung mit den Spulenelementen empfangen wurden, und/oder
• einer Auswertung eines Videosignals, das mit zumindest einer Kamera aufgenommen wurde, und/oder
• einer Auswertung eines Sensorsignals, das mit zumindest einem Positionssensor erfasst wurde.

8. Verfahren nach einem der vorangehenden Ansprüche,
wobei die Auswahl der ersten Menge von Spulenelementen und/oder der zweiten Menge von Spulenelementen vollautomatisch und/oder halbautomatisch und/oder manuell erfolgt, wobei bei einer vollautomatischen Auswahl von Spulenelementen ein Bedienpersonal der Magnetresonanzvorrichtung an der Auswahl der Spulenelemente nicht mitwirkt,
wobei bei einer halbautomatischen Auswahl dem Bedienpersonal eine Menge von Spulenelementen automatisch vorgeschlagen wird und das Bedienpersonal basierend auf diesem Vorschlag die Spulenelemente auswählt.

9. Verfahren nach einem der vorangehenden Ansprüche,
wobei die Auswahl der zweiten Menge an Spulenelementen erfolgt anhand
• eines Typs zumindest einer Empfangsspule, welche zumindest einen Teil der Mehrzahl von Spulenelementen der Magnetresonanzvorrichtung umfasst, und/oder
• einer relativen Lage der Spulenelemente innerhalb zumindest einer Empfangsspule, welche zumindest einen Teil der Mehrzahl von Spulenelementen der Magnetresonanzvorrichtung umfasst, und/oder
• zumindest einer Eigenschaft des Patienten und/oder
• einer Positionierung des Patienten in der Magnetresonanzvorrichtung, insbesondere auf einer Patientenliege.

10. Verfahren nach einem der vorangehenden Ansprüche,
wobei mit zumindest einem Teil der Mehrzahl der Spulenelementen der Magnetresonanzvorrichtung jeweils ein Justage-Pilottonsignal aufgenommen werden,
wobei die Justage-Pilottonsignale hinsichtlich ihres jeweiligen Beitrags zur Ermittlung der Bewegungsinformation des Patienten ausgewertet werden,
wobei eine maximale Anzahl K der Spulenelemente der zweiten Menge von Spulenelementen ermittelt wird,
wobei K Spulenelemente ausgewählt und/oder vorgeschlagen werden, die den größten Beitrag zur Ermittlung der Bewegungsinformation des Patienten aufweisen.

11. Verfahren nach Anspruch 10,
wobei der zumindest eine Teil der Mehrzahl der Spulenelemente der Magnetresonanzvorrichtung, mit dem jeweils ein Justage-Pilottonsignal aufgenommen wird, bestimmt wird anhand
• eines Typs zumindest einer Empfangsspule, welche zumindest einen Teil der Mehrzahl von Spulenelementen der Magnetresonanzvorrichtung umfasst, und/oder
• einer relativen Lage der Spulenelemente innerhalb zumindest einer Spule, welche zumindest einen Teil der Mehrzahl von Spulenelementen der Magnetresonanzvorrichtung umfasst, und/oder
• zumindest einer Eigenschaft des Patienten und/oder
• einer Positionierung des Patienten in der Magnetresonanzvorrichtung, insbesondere auf einer Patientenliege.

12. Verfahren nach einem der vorangehenden Ansprüche,
wobei zur Auswahl der Spulenelemente einem Bedienpersonal ein Avatar des Patienten und dazu lagerichtig zumindest ein Teil der Mehrzahl von Spulenelementen der Magnetresonanzvorrichtung angezeigt wird,
wobei ferner angezeigt wird, ob ein angezeigtes Spulenelement zur ersten Menge von Spulenelementen und/oder zur zweiten Menge von Spulenelemente zugeordnet ist.

13. Verfahren nach Anspruch 12,
wobei dem Bedienpersonal zur Auswahl der Spulenelemente ferner ein Bildgebungsbereich angezeigt wird.

14. Magnetresonanzvorrichtung mit einer Mehrzahl an Spulenelementen, die ausgebildet ist, ein Verfahren nach einem der vorangehenden Ansprüche auszuführen.

15. Computerprogrammprodukt, welches ein Programm umfasst und direkt in einen Speicher einer programmierbaren Systemsteuereinheit einer Magnetresonanzvorrichtung ladbar ist, mit Programmmitteln, um ein Verfahren nach einem der Ansprüche 1 bis 13 auszuführen, wenn das Programm in der Systemsteuereinheit der medizinischen Bildgebungsvorrichtung ausgeführt wird.
